# EUROPEAN PATENT SPECIFICATION

(11) **EP 3 258 831 B1**
(45) Date of publication and mention of the grant of the patent: **24.05.2023**
(21) Application number: 16708098.5
(22) Date of filing: 22.02.2016
(51) Int. Cl.: A61B 5/00, A61B 10/00, A61K 39/35

(54) **INFANT SKIN TEST DEVICE AND METHODS FOR USING SAME**
HAUTTESTVORRICHTUNG FÜR KINDER UND VERFAHREN ZUR VERWENDUNG DAVON
DISPOSITIF DE TEST CUTANÉ POUR NOURRISSON ET PROCÉDÉS D'UTILISATION ASSOCIÉS

(30) Priority: 20.02.2015 US 201562118875 P
(43) Date of publication of application: 27.12.2017
(73) Proprietor: Société des Produits Nestlé S.A., 1800 Vevey (CH)
(72) Inventor: BLANCHARD, Carine, 1052 Le Mont-sur-Lausanne (CH)
(74) Representative: Elkington and Fife LLP
(86) International application number: PCT/EP2016/053673
(87) International publication number: WO 2016/131988

(56) References cited:
- WO-A1-2014/125485
- WO-A2-2007/059979
- US-A- 4 818 707
- US-A1- 2007 048 361
- US-A1- 2014 276 196
- DATABASE BIOSIS [Online] BIOSCIENCES INFORMATION SERVICE, PHILADELPHIA, PA, US; 30 April 2010 (2010-04-30), DUPONT C ET AL: "Atopy Patch Test for Early Diagnosis of Cow's-Milk Allergy in Preterm Infants", Database accession no. PREV201000245730 & JPGN JOURNAL OF PEDIATRIC GASTROENTEROLOGY AND NUTRITION, vol. 50, no. 4, April 2010 (2010-04), pages 463-464, ISSN: 0277-2116, DOI: 10.1097/MPG.0B013E3181B97BED

## Description

### BACKGROUND

The present disclosure relates generally to a device for detecting an individual's immune responses. More specifically, the present disclosure relates to skin patch devices for detecting an infant's immune response to different infant formulas. Additionally, the present disclosure relates to skin patch devices for detecting an infant's immune response to protein components having varying degrees of hydrolysis.

Cows' milk protein allergy ("CMPA") is the most frequently occurring food allergy in infants. Two forms of CMPA can occur: a IgE mediated response that leads to immediate symptoms, and a non-IgE mediated response that leads to delayed symptoms in response to the milk proteins that are normally harmless to the non-allergic, tolerant individual. In most infants with CMPA, it is usually a non-IgE mediated response that is exhibited. Non-IgE mediated CMPA is mediated by T-lymphocytes, and is not detectable by allergy blood tests quantifying IgE levels. Because it is not caused by antibodies, it can take several hours, or even up to 72 hours to produce a clinical effect. This makes diagnosis of CMPA rather difficult for healthcare providers.

Additionally, CMPA can produce a range of often uncomfortable gastrointestinal and dermatological symptoms for infants suffering from such an allergy. Gastrointestinal symptoms may include vomiting, infantile colic, gastroesophageal reflux, oesophagitis, diarrhea, constipation, stomach pain, or flatulence. In some cases, particularly infants, gastrointestinal symptoms may also include blood and/or mucus in the stool. Atopic dermatitis is a common dermatological symptom. Because most commercial infant formula brands are based on cows' milk proteins, it is important to diagnose CMPA allergies in infants early to avoid such symptoms and to prevent any additional medical conditions that may result from the allergy (e.g., failure to thrive).

One method that medical professionals use to diagnose CMPA is a skin patch test. Known skin patch tests, however, are configured merely for the detection of CMPA, and are not able to determine an infant's level of allergic response severity. Accordingly, it can be a long and trying process to determine which, of many different brands and types, infant formula is best suited for an infant with a known CMPA. During this trial period, infants may continue to exhibit many of the symptoms disclosed above, which results in an uncomfortable and fussy baby, and exhausted and frustrated caretakers.

Skin patch test devices for determining allergies to food substances are known. US4818707A relates to a skin patch device for testing an individual's Gell Coombs Types I, II, III and IV immune responses to edible substances. These skin patch devices allow a much faster and less expensive determination of allergens to food. However, there still remains a need for a device which can determine the level of an allergic response.

### SUMMARY

Applicants have developed skin test devices that are particularly useful in stratification of patients to infant formulas and protein components with different levels of allergenicity. In this regard, the skin test devices of the present disclosure can serve as an assignment tool to help healthcare providers and caretakers determine which infant formulas are most physiologically compatible with infants having a cow's milk protein allergy. Such a skin test device is useful not only in avoiding the long, physically uncomfortable process of trial and error in determining an infant formula that is best suited for an infant, but also in avoiding the increased cost to consumers of infant formulas that contain proteins that are extensively hydrolyzed or infant formulas that are amino acid-based. Indeed, it is better physiologically for an infant to be fed a hydrolyzed formula than an amino acid-based formula that does not contain any peptides. Some healthcare providers, however, do not follow current guidelines for formula assignment and instead assign amino acid-based formula to a majority of patients to ensure a positive result.

Accordingly, in a general embodiment, the present disclosure provides a skin test device comprising at least three chambers, characterised in that, a first chamber houses a negative control material, a second chamber houses extensively hydrolyzed protein infant formula or a partially hydrolyzed protein infant formula, and a third chamber houses an intact casein and whey protein infant formula..

In an embodiment, the infant formula is an infant formula having a protein component that is selected from the group consisting of intact casein protein, intact whey protein, extensively hydrolyzed protein (either casein or whey), partially hydrolyzed protein (either casein or whey), amino acid, or combinations thereof.

In an embodiment, the skin test device is a single, integral device that has a plurality of chambers.

In an embodiment, the skin test device is a kit comprising a skin test device comprising at least three chambers, characterised in that, a first chamber houses a negative control material, a second chamber houses extensively hydrolyzed protein infant formula or a partially hydrolyzed protein infant formula, and a third chamber houses an intact casein and whey protein infant formula.

In an embodiment, the skin test device includes at least five individual chambers. At least four chambers each house a different infant formula and/or protein component with respect to each other, and a negative control (exempt of nitrogen, for example saline). For example, in an embodiment, a first chamber has saline, a second chamber has an extensively hydrolyzed protein infant formula, a third chamber has an intact casein and whey protein infant formula, a fourth chamber has a partially hydrolyzed protein infant formula, and a fifth chamber has an amino acid-basedinfant formula.

In an embodiment, the one of a negative control material, an infant formula and a protein component is provided in a form selected from the group consisting of a liquid, a solid, a paste, a powder, a gel, or combinations thereof.

In an embodiment, the skin test device further includes a substance housed by the chamber, the substance being impregnated or coated with the one of a negative control material, an infant formula and a protein component.

In another embodiment, a skin test device is provided. The skin test device includes a substrate or support that forms at least a portion of each of a plurality of cells. Each cell includes a chamber, a negative control material housed in a chamber of at least one of the plurality of cells, and a plurality of materials to be tested for the occurrence of a local allergic reaction on an infant's skin. At least two of the plurality of materials are (i) different materials, (ii) housed in separate chambers, and (iii) one of an infant formula and a protein component selected from the group consisting of an amino acid, a hydrolyzed protein, intact casein protein, intact whey protein, or combinations thereof. The hydrolyzed protein may be one of extensively hydrolyed protein and partially hydrolyzed protein.

In an embodiment, the skin test device has at least five individual cells, each cell including a chamber. At least four chambers each house a different infant formula and/or protein component with respect to each other. For example, in an embodiment, a first chamber has saline, a second chamber has an an extensively hydrolyzed protein infant formula, a third chamber has an intact casein and whey protein protein infant formula, a fourth chamber has a partially hydrolyzed protein infant formula, and a fifth chamber has an amino acid-based infant formula.

In an embodiment, the one of a negative control material, an infant formula and a protein component is provided in a form selected from the group consisting of a liquid, a solid, a paste, a powder, a gel, or combinations thereof.

In an embodiment, the skin test device further includes a substance housed by the chamber, the substance being impregnated or coated with the one of a negative control material, an infant formula and a protein component.

In an embodiment, the skin test device includes at least three individual chambers. In an embodiment, the at least three chambers each house a different infant formula and/or protein component with respect to each other. For example, a first chamber may include saline, a second chamber may include an extensively hydrolyzed protein infant formula, and a third chamber has an intact casein and whey protein infant formula. In another embodiment, a first chamber may include a negative control (e.g. saline), a second chamber may include a partially hydrolyzed protein infant formula, and a third chamber has an intact casein and whey protein infant formula.

In yet another embodiment, a kit for skin testing is provided. The kit includes a skin test device having at least three chambers, characterised in that, a first chamber houses a negative control material, a second chamber houses extensively hydrolyzed protein infant formula or a partially hydrolyzed protein infant formula, and a third chamber houses an intact casein and whey protein infant formula. The kit further includes instructions for applying the skin test device to the skin of an infant.

In an embodiment, the instructions include at least one of removing a removable cover from the skin test device, adhering the skin test device to the skin of the infant, and allowing the skin test to remain adhered to the skin of the infant for a predetermined amount of time.

In an embodiment, the skin test device further includes a substance housed by each of the plurality of chambers. The substance may be impregnated or coated with the one of a negative control material, an infant formula and a protein component.

In an embodiment, a first chamber has saline, a second chamber comprises an extensively hydrolyzed protein infant formula, a third chamber comprises an intact casein and whey protein infant formula, a fourth chamber comprises a partially hydrolyzed protein infant formula, and a fifth chamber comprises an amino acid-based infant formula.

In still yet another embodiment, a method for using a skin test device is provided. The method includes providing a parent or caretaker of the infant with a skin test device that has at least three chambers, characterised in that, a first chamber houses a negative control material, a second chamber houses extensively hydrolyzed protein infant formula or a partially hydrolyzed protein infant formula, and a third chamber houses an intact casein and whey protein infant formula. The method further includes instructing the parent or caretaker to perform a skin patch test by adhering the skin test device to the skin of the infant for a predetermined amount of time.

In an embodiment, the method may further include instructing the parent to schedule an appointment with a healthcare provider to read a result of the skin patch test after the predetermined amount of time. The healthcare provider can then assign an infant formula to the infant based on the result.

In an embodiment, the method may further include instructing the parent to remove the skin test device after the predetermined amount of time. The method may also include instructing the parent to read a result of the skin patch test after removal of the skin test device. After reading the result, a healthcare provider can then assign an infant formula to the infant based on the result.

An advantage of the present disclosure is to provide a skin test device that helps to determine an infant's tolerance for a variety of infant formulas.

Another advantage of the present disclosure is to provide a skin test device that helps to determine an infant's tolerance for different protein hydrolysates.

Still another advantage of the present disclosure is to provide a multi-chamber patch test device that allows healthcare providers and/or caretakers to select an infant formula for an infant based upon the severity of the infant's cow's milk protein allergy.

An additional advantage of the present disclosure is to provide an assignment tool to help healthcare providers and/or caretakers cost-effectively select an allergenically compatible infant formula for an infant.

Another advantage of the present disclosure is to provide a kit that allows for application of a skin test device for an infant by a medical professional or a caretaker.

Yet another advantage of the present disclosure is to provide methods for stratifying infants to infant formulas with different levels of allergic response.

Another advantage of the present disclosure is to provide methods for assigning an infant to an allergenically compatible infant formula.

Additional features and advantages are described in, and will be apparent from, the following Detailed Description and the Figures.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
**FIG. 1** illustrates a skin test device in accordance with an embodiment of the present disclosure.
**FIG. 2** illustrates an exploded, perspective view of a skin test device cell in accordance with an embodiment of the present disclosure.
**FIG. 3** illustrates a cross-sectional view, taken generally along line III-III of the skin test device of FIG. 1.
**FIG. 4** illustrates an exemplary configuration of a skin test device in accordance with an embodiment of the present disclosure.
**FIG. 5** illustrates another exemplary configuration of a skin test device in accordance with an embodiment of the present disclosure.
**FIG. 6** illustrates a graphical representation of a patch of skin subjected to a skin test device in accordance with an embodiment of the present disclosure wherein the patch of skin indicates a tolerance for infant formula that includes intact casein and whey protein.
**FIG. 7** illustrates a graphical representation of a patch of skin subjected to a skin test device in accordance with an embodiment of the present disclosure wherein the patch of skin indicates a tolerance for infant formula that includes extensively hydrolyzed protein.
**FIG. 8** illustrates a graphical representation of a patch of skin subjected to a skin test device in accordance with an embodiment of the present disclosure wherein the patch of skin indicates a tolerance for infant formula that includes amino acids.
**FIG. 9** depicts a patch of skin subjected to a skin test device in accordance with an embodiment of the present disclosure wherein the patch of skin indicates an absence of reaction to a skin test allergen.
**FIG. 10** depicts a patch of skin subjected to a skin test device in accordance with an embodiment of the present disclosure wherein the patch of skin indicates a weak positive reaction to a skin test allergen.
**FIG. 11** depicts a patch of skin subjected to a skin test device in accordance with an embodiment of the present disclosure wherein the patch of skin indicates a strong positive to a skin test allergen.
**FIG. 12** depicts a patch of skin subjected to a skin test device in accordance with an embodiment of the present disclosure wherein the patch of skin indicates an extreme reaction to a skin test allergen.

### DETAILED DESCRIPTION

All percentages are by weight of the total weight of the composition unless expressed otherwise. Similarly, all ratios are by weight unless expressed otherwise. When reference is made to the pH, values correspond to pH measured at 25 °C with standard equipment.

Furthermore, all numerical ranges herein should be understood to include all integers, whole or fractions, within the range. Moreover, these numerical ranges should be construed as providing support for a claim directed to any number or subset of numbers in that range. For example, a disclosure of from 1 to 10 should be construed as supporting a range of from 1 to 8, from 3 to 7, from 1 to 9, from 3.6 to 4.6, from 3.5 to 9.9, and so forth.

As used herein and in the appended claims, the singular form of a word includes the plural, and vice versa, unless the context clearly dictates otherwise. Thus, the references "a," "an" and "the" are generally inclusive of the plurals of the respective terms. For example, reference to "an ingredient" or "a method" includes a plurality of such "ingredients" or "methods." The term "and/or" used in the context of "X and/or Y" should be interpreted as "X," or "Y," or "X and Y."

As used herein, "about" is understood to refer to numbers in a range of numerals, for example the range of -10% to +10% of the referenced number, preferably -5% to +5% of the referenced number, more preferably -1% to +1% of the referenced number, most preferably -0.1 % to +0.1% of the referenced number.

Similarly, the words "comprise," "comprises," and "comprising" are to be interpreted inclusively rather than exclusively. Likewise, the terms "include," "including" and "or" should all be construed to be inclusive, unless such a construction is clearly prohibited from the context. However, the embodiments provided by the present disclosure may lack any element that is not specifically disclosed herein. Thus, a disclosure of an embodiment defined using the term "comprising" is also a disclosure of embodiments "consisting essentially of' and "consisting of' the disclosed components. Where used herein, the term "example," particularly when followed by a listing of terms, is merely exemplary and illustrative, and should not be deemed to be exclusive or comprehensive. Any embodiment disclosed herein can be combined with any other embodiment disclosed herein unless explicitly indicated otherwise.

As used herein, "complete nutrition" contains sufficient types and levels of macronutrients (protein, fats and carbohydrates) and micronutrients to be sufficient to be a sole source of nutrition for the animal to which the composition is administered. Individuals can receive 100% of their nutritional requirements from such complete nutritional compositions.

The terms "food," "food product" and "food composition" mean a product or composition that is intended for ingestion by a human and provides at least one nutrient to the human. The compositions of the present disclosure, including the many embodiments described herein, can comprise, consist of, or consist essentially of the essential elements and limitations described herein, as well as any additional or optional ingredients, components, or limitations described herein or otherwise useful in a diet for elderly males.

The term "infant" is used herein to mean a child under the age of 12 months.

The term "infant formula" as used herein refers to a nutritional formulation (either in the form of a liquid or in the form of a dry powder that may be reconstituted to form a liquid infant formula upon addition of water) that provides complete nutrition for an infant and may be used as a substitute for human milk in feeding an infant. Such formulas are well-known in the art.

As used herein the term "patient" is understood to include an animal, especially a mammal, and more especially a human that is receiving or intended to receive treatment, as it is herein defined. In an embodiment, a patient of the present disclosure is an infant or a toddler.

The terms "treatment" and "treating" include any effect that results in the improvement of the condition or disorder, for example lessening, reducing, modulating, or eliminating the condition or disorder. Non-limiting examples of "treating" or "treatment of" a condition or disorder include: (1) inhibiting the condition or disorder, i.e. arresting the development of the condition or disorder or its clinical symptoms and (2) relieving the condition or disorder, i.e. causing the temporary or permanent regression of the condition or disorder or its clinical symptoms.

As discussed above, cows' milk protein allergy ("CMPA") is the most frequently occurring food allergy in infants. Two forms of CMPA can occur: a IgE mediated response that leads to immediate symptoms, and a non-IgE mediated response that leads to delayed symptoms in response to the milk proteins that are normally harmless to the non-allergic, tolerant individual. In most infants with CMPA, it is usually a non-IgE mediated response that is exhibited. Non-IgE mediated CMPA is mediated by T-lymphocytes, and is not detectable by allergy blood tests quantifying IgE levels. Because it is not caused by antibodies, it can take several hours, or even up to 72 hours to produce a clinical effect. This makes diagnosis of CMPA rather difficult for healthcare providers.

Unfortunately, CMPA can produce a range of often uncomfortable gastrointestinal and dermatological symptoms for infants suffering from such an allergy. Gastrointestinal symptoms may include vomiting, and infantile colic, gastroesophageal reflux, oesophagitis, diarrhea, constipation, stomach pain, or flatulence. In some cases, particularly infants, gastrointestinal symptoms may also include blood and/or mucus in the stool. Atopic dermatitis is a common dermatological symptom. Because most commercial infant formula brands are based on cows' milk proteins, it is important to diagnose CMPA allergies in infants early to avoid such symptoms and to prevent any additional medical conditions that may result from the allergy (e.g., failure to thrive).

Treatment for CMPA is very similar to that for antibody-mediated milk allergy. One method that medical professionals use to diagnose CMPA is a skin patch test. A patch test relies on the principle of a type IV hypersensitivity reaction and may identify allergens not identified by alternative allergy testing methods including, for example, blood testing or skin prick testing. Patch testing is used to test for the occurrence of a local allergic reaction on a small area of the patient's skin (usually the back), where the expected allergens were previously placed. In this regard, the patch test response is merely contact dermatitis in a small area.

Application of a patch test device usually takes up to about half an hour. During that time, tiny quantities of, for example, about 25 to about 150 materials (allergens) in individual square plastic or round aluminium chambers are applied to the upper back and are kept in place with special hypoallergenic adhesive tape, or other stabilizing means. The patches stay in place undisturbed for a predetermined amount of time. In general, it takes about 2 to about 4 days for a response in patch testing to develop.

After the predetermined amount of time, the patches are removed by either the patient, a caretaker, or a healthcare provider. The skin is usually marked to identify the test sites, and a preliminary reading is done. After about 24 to about 48 hours after test site marking, a final reading is completed (i.e., about 72 to about 96 hours after application) to determine which test materials induced an allergic reaction on the patient's skin. In some cases, an additional reading or two may be requested by a healthcare provider.

Known skin patch tests, however, are configured merely for the detection of CMPA, and are not able to determine an patient's level of allergic response to an infant formula or to several infant formulas. This is especially problematic when the patient is an infant that is experiencing an uncomfortable allergic reaction to an infant formula or a protein component of an infant formula that is administered to the infant several times each day. It can be a long and trying process to determine which, of many different brands and types, infant formula is best suited for an infant with a known CMPA. During this trial period, infants may continue to exhibit many of the symptoms disclosed above, which results in an uncomfortable and fussy baby, and exhausted and frustrated caretakers. Accordingly, it would be beneficial for caretakers and/or healthcare providers to be able to test infants with CMPA for varying degrees of allergic response to assign infants to an infant formula for which they are best suited.

Applicants have, therefore, developed a skin patch test that may be used in a multi-chamber format with a single, yet different infant formula (or protein component) in each chamber in order to compare different infant formulas and assign the patient/infant to the appropriate infant formula. In this manner, the patch tests allow for stratification of patients to infant formula with different levels of allergic response, such as different infant formulas with different extents of protein hydrolysis. Indeed, the more extensively hydrolyzed the protein, the less allergenic the formula is expected to be. For example, an infant formula that is amino acid-based would be expected to be a very low allergenic formula and would be assigned to infants with severe CMPA. Alternatively, an infant formula that is based on intact casein and whey protein would be expected to be a high allergenic formula and would be assigned only to infants with very mild, or non-existant CMPA.

Such stratification is beneficial not only for expedient diagnosis of CMPA and proper assignment of infant formula so as to avoid physiological complications (e.g., gastrointestinal symptoms, dermatological symptoms, failure to thrive, etc.), but also from a cost perspective. In this regard, amino acid-based infant formulas require assembly of a single amino acid and are extremely costly when conmpared to infant formulas that are based on intact casein or whey protein. As a result, amino acid-based infant formulas are much more expensive for caretakers. Due to the higher cost of the amino acid-based formula it is beneficial to be able to assign infants a less hydrolyzed (i.e., less expensive) infant formula if the child can tolerate the formula without developing symptoms associated with CMPA.

Accordingly, the present disclosure is directed to a single, ready-to-use skin test device including at least threechambers in which different types of infant formulas and/or protein components may be placed. The skin test device is used not only for diagnosis of CMPA, but also for detection of a degree of allergic response of an infant with respect to specific infant formulas and/or protein hydrolysates. The skin test devices of the present disclosure, therefore, are able to assign an infant with the most cost-efficient and physiologically compatible infant formula available so as to avoid unnecessary symptoms or medical conditions associated with CMPA, as well as overspending by infant caretakers.

The skin test devices of the present disclosure may be applied by either a healthcare professional (e.g., a doctor, a nurse, etc.) or a parent/caretaker. Accordingly, the skin test devices may be (i) applied by a healthcare professional and the results of the test read by either the healthcare professional or a parent/caretaker at the end of the predetermined duration of the test, or (ii) applied by a parent/caretaker and the results of the test read by either a healthcare professional or the parent/caretaker at the end of the predetermined duration of the test. The test results may be read by the parent/caretaker in the home, or by a healthcare professional at an appointment at a healthcare facility.

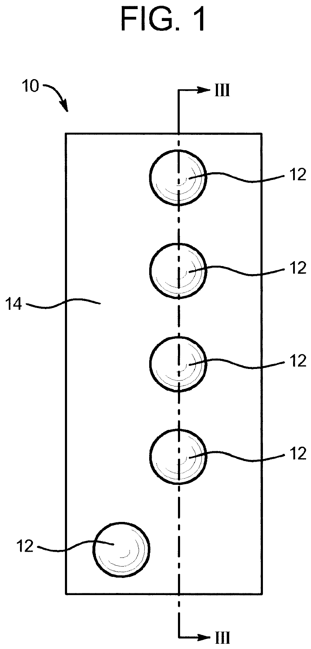

A single skin test device of the present disclosure includes a plurality of cells therein that each form a chamber that may contain a different material to be tested for the occurrence of a local allergic reaction on a patient's skin. For example, and as shown in FIG. 1, a skin test device 10 may include a plurality of cells 12 formed at least partially by a support 14. Each cell 12 is so constructed and arranged to house at least one of a negative control material, a positive control material and a material to be tested for the occurrence of a local allergic reaction on a patient's skin. In this regard, cells 12 may be generally pillow-shaped or hemispherically-shaped in able to form a chamber in which the materials are housed.

The plurality of cells 12, if designed as autoadesive can be applied on the skin alone. In that case support 14 can be one, or a plurality of, plastic, hard or soft applicator(s) that may be thrown away after adhesion of the plurality of cells 12 on the skin to leave on the skin only the three to five cells 12 without the need for support 14 to adhere on the skin.

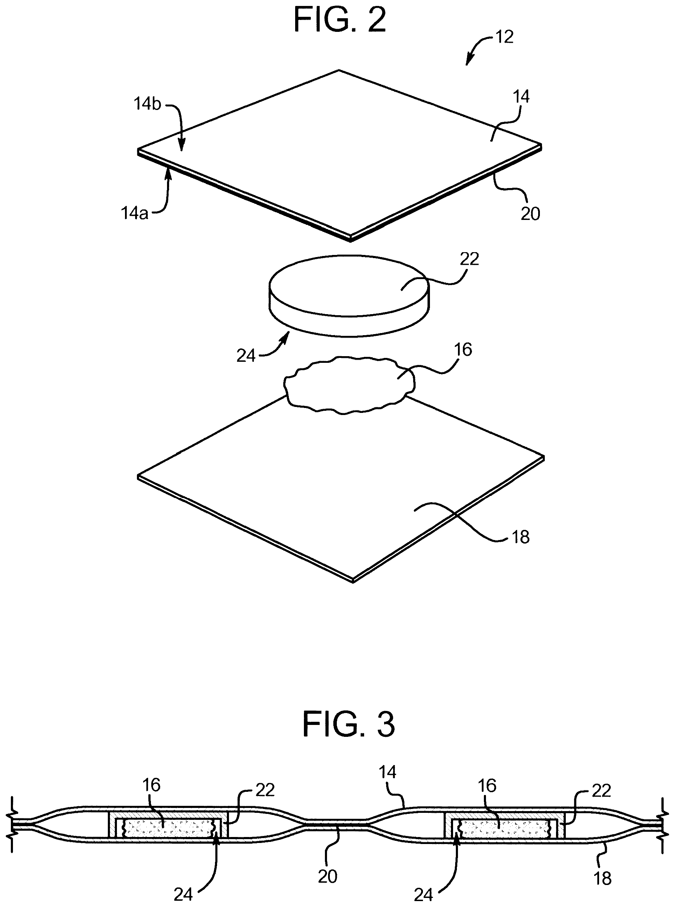

For example, FIG. 2 illustrates an exemplary single cell 12 of a skin test device 10 of the present disclosure. As shown in FIG. 2, cell 12 includes support 14 and a material 16 to be tested for the occurrence of a local allergic reaction on the patient's skin. Prior to use, support 14 and material 16 may be protected by a removable cover 18, which also aids in maintaining materials 16 in each individual cell 12 during shipping and/or storage.

Support 14 may be provided with an adhesive coating 20 on a bottom side 14a that is intended to be adhered to a patient's skin. In this regard, once removable cover 18 is removed for use, skin test device 10 may be adhered to a patient's skin using adhesive coating 20, which may be a pressure-sensitive adhesive. Adhesive coating 20 may be applied to the entire bottom side 14a of support 14, or may be applied only to portions of bottom side 14a of support 14 between cells 12. Support 14 may be, for example, any appropriate adhesive tape, or any appropriately flexible and soft material that is substantially non-toxic and inert. Support 14 may also be rigid enough to form integral chambers therein, as will be discussed below. Regardless, support 14 includes a plurality of cells 12 for the testing of several materials 16 at one time, in addition to at least one of a positive and negative control material.

As mentioned previously, support 14 may be rigid enough to form by itself a chamber in which material 16 is housed. In this embodiment, support 14 has integrally formed chambers (not shown) that are convex in shape and protrude upward from a top surface 14b of support 14. Alternatively, support 14 may simply be made of a sufficiently flexible material that deforms to the shape of a housing 22 that is so constructed and arranged to house material 16. In this embodiment, housing 22 may have a shape that is substantially cylindrical and hollow such that housing 22 can form a chamber 24 in which material 16 is housed.

To better demonstrate the structure of a skin test device of the present disclosure, FIG. 3 shows a cross-sectional view of the skin test device illustrated in FIG. 1. In this embodiment, support 14 is made of a sufficiently flexible material that deforms to the shape of housing 22, which forms a chamber 24 in which material 16 is housed. When cover 18 is removed, support 14 is adhered to a patient's skin and material 16 is kept in contact with the patient's skin for the duration of the patch test.

Material 16 may be provided in a variety of forms selected from the group consisting of a liquid, a solid, a paste, a powder, a gel, or combinations thereof. In this regard, material 16 may be a liquid solution that is expected to produce an allergic reaction in a patient having CMPA. In another embodiment, material 16 may be a powder that is expected to produce an allergic reaction in a patient having CMPA. In yet another embodiment, material 16 may be a substance that is impregnated or coated with a liquid, solid, paste, powder, gel, etc. that is expected to produce an allergic reaction in a patient having CMPA. A positive control material may be any material that is expected to produce an allergic reaction in a patient having CMPA. For example, the positive control material may be an infant formula that includes intact casein and whey protein. The negative control material may be, for example, saline.

The materials to be tested for the occurrence of an allergic response in a patient (e.g., an individual having CMPA, or the like) may include, but are not limited to, infant formulas having intact casein protein, infant formulas having intact whey protein, infant formulas having partially hydrolyzed whey protein, infant formulas having extensively hydrolyzed whey protein, infant formulas having partially hydrolyzed casein, infant formulas having extensively hydrolyzed casein, infant formulas having amino acids, infant formulas having plant protein or plant protein hydrolyzate, infant formulas having rice or rice hydrolyzate, infant formulas having egg, infant formulas having egg hydrolyzate, and the like. Alternatively, the materials to be tested for the occurrence of an allergic response in a patient might not be infant formulas and may simply include, but are not limited to, intact casein, intact whey protein, partially hydrolyzed whey protein, extensively hydrolyzed whey protein, partially hydrolyzed casein, extensively hydrolyzed casein, amino acids, rice, egg, rice hydrolyzate, egg hydrolyzate, plant protein or plant protein hydrolyzate and the like.

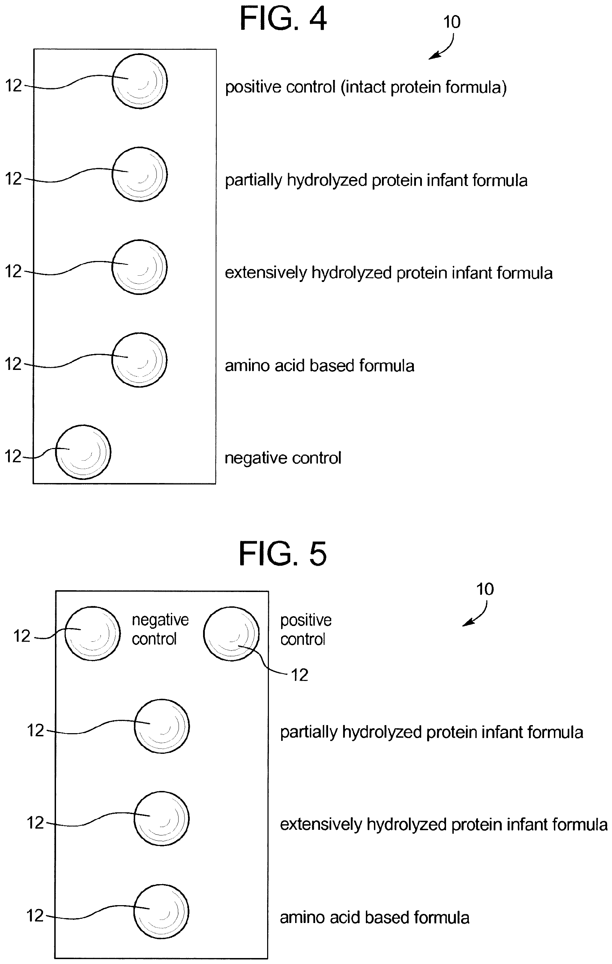

The skilled artisan will appreciate that the skin test devices of the present disclosure need not be limited to any specific configuration. Accordingly, FIGS. 4 and 5 illustrate two different configurations of skin test devices. In FIG. 4, for example, the top four cells 12 contain materials 16 to be tested for the occurrence of a local allergic reaction on the patient's skin, while the bottom, offset cell 12 contains a negative control. In FIG. 4, the material expected to produce a local allergic reaction and, thereby serving as a positive control, is an infant formula containing intact casein and whey protein. A second cell 12 houses a material that is a partially hydrolyzed infant formula. A third cell 12 houses a material that is an extensively hydrolyzed infant formula. A fourth cell 12 houses a material that is an amino acid-based infant formula. Again, the material may be, for example, a liquid infant formula, a powder infant formula, a substance impregnated with an infant formula, a substance coated with an infant formula, etc. Alternatively, the material may be, for example, a partially hydrolyzed protein, an extensively hydrolyzed protein, an amino acid, a substance impregnated with an extensively hydrolyzed protein, a substance coated with an extensively hydrolyzed protein, etc.

The skilled artisan will also appreciate that, although the skin test devices of the present disclosure are illustrated as having five cells each, the devices may include more or less cells, depending on the skin testing needs. For example, if an infant were being tested for allergic responses to six different infant formulas, a skin test device may include, for example, seven cells, which provides an additional cell for a negative control material.

FIG. 5 illustrates a second exemplary configuration of a skin test device 10. In this embodiment, cells 12 containing the negative and positive controls are located at a top of the skin test device 10, while the three cells 12 containing test materials are located under cells 12 containing the negative and positive controls. Although FIGS. 4 and 5 are illustrated as having cells with materials that have a descending order of protein hydrolysis (i.e., the least hydrolyzed protein formula is near the top and the most hydrolyzed formula is near the bottom), the skilled artisan will appreciate that cells 12 may contain any materials in any desired order or configuration.

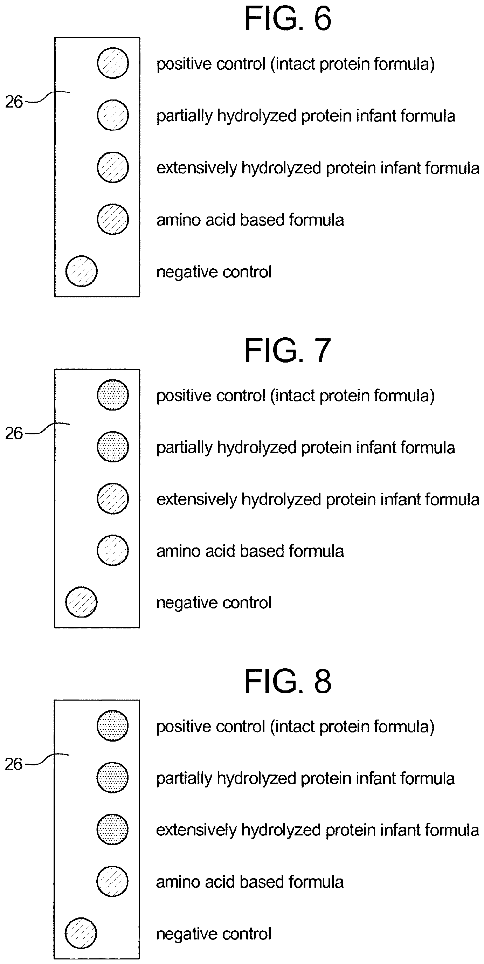

Once the duration of the skin patch test is completed, the skin test device may be removed from the patient's skin and the results may be interpreted by a healthcare provider or a parent/caretaker, for example 24 to 72 hours after removal . FIGS. 6-8 illustrate graphical representations of patches 26 of a patient's skin that have been exposed to skin test devices of the present disclosure. For example, as shown in FIG. 6, a patient's skin that was exposed to the skin test device 10 of FIG. 4 shows no allergic reaction (i.e., cross-hatching is a graphical representation of normal skin), which indicates that the patient is tolerant of all tested infant formulas contained in skin test device 10 of FIG. 4. Specifically, the patient's skin patch 26 indicates that the patient is tolerant of the least hydrolyzed protein based infant formula (i.e., intact protein formula). Accordingly, a healthcare provider or a caretaker could assign the infant to the least hydrolyzed infant formula tested, which would typically be the most cost-effective infant formula.

As shown in FIG. 7, a patient's skin patch 26 that was exposed to the skin test device 10 of FIG. 4 shows a postive reaction to both the positive control and the partially hydrolyzed infant formula (i.e., sipple pattern is a graphical representation of an allergic reaction on the skin), but shows no reaction to the extensively hydrolyzed infant formula or the amino acid-based infant formula. Accordingly, a healthcare provider or a caretaker could assign the infant to the extensively hydrolyzed infant formula, which would typically be more cost-effective than the amino acid-based infant formula.

As shown in FIG. 8, a patient's skin patch 26 that was exposed to the skin test device 10 of FIG. 4 shows a positive reaction to the positive control, the partially hydrolyzed infant formula and the extensively hydrolyzed infant formula (i.e., sipple pattern is a graphical representation of an allergic reaction on the skin), but shows no reaction to the amino acid-based infant formula. Accordingly, a healthcare provider or a caretaker could assign the infant to the amino acid-based infant formula, which, unfortunately, is typically the most costly infant formula.

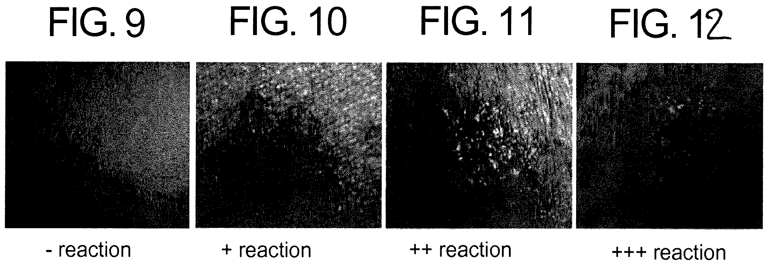

FIGS. 9-12 depict actual skin patch reactions to demonstrate the variance of expected skin reactions. For example, FIG. 9 shows an equivocal/uncertain (+/-) reaction to a material expected to produce a local allergic reaction on the patient's skin; FIG. 10 shows a weak positive reaction to a material expected to produce a local allergic reaction on the patient's skin; FIG. 11 shows a strong positive reaction to a material expected to produce a local allergic reaction on the patient's skin; and FIG. 12 shows an extreme reaction to a material expected to produce a local allergic reaction on the patient's skin. Typically, weak positive reactions are slightly elevated pink or red plaques, usually with mild vesiculation. Strong positive reactions are usually 'papulovesicles,' and extreme reactions usually have spreading redness, severe itching, and blisters or ulcers.

In an alternate embodiment, instead of reading the test results on the skin of the test subject after removal of the skin test device, the device may be impregnated with a substance that will turn the device a specific color if the test subject exhibits an allergic response. For example, material 16 may be impregnated with a substance that will turn blue when the skin of the test subject begins to exhibit an allergic response. In this manner, it may be possible to determine whether a test subject responds to some, if not all, of the materials 16 in the chambers 24, even without removing the skin test device.

Since the present skin test devices does not need to be applied in a medical facility and may, for example, be applied by a parent or caretaker at home, the present disclosure also provides for kits containing at least one skin test device 10 in combination with instructions for use of the skin test device 10. In this regard, a kit may include any skin test device 10 described herein in combination with instructions for use of same. The instructions may be written instructions contained on, for example, a pamphlet or paper in the kit and may include a number of steps for use of the skin test device 10. For example, the steps may recite removing a protective cover, applying a skin test device to the patient's skin (e.g., an infant's back), leaving the skin test device on the patient's skin for a predetermined amount of time, and removing the skin test device after the expiration of the predetermined amount of time. The predetermined amout of time may be, for example, between about 24 hours and about 96 hours. In an embodiment, the predetermined amount of time is about 24 hours. In an embodiment, the predetermined amount of time is about 48 hours.

The instructions may also include, for example, a step requiring the user (e.g., caretaker, doctor, nurse, etc.) to made a doctor appointment to have the skin test device removed and the skin patch results read by a healthcare provider, a step of documenting the skin patch results for interpretation by a healthcare provider (e.g., a hand-drawn illustration, a color photograph, etc.), a step for removing the skin test device earlier than the predetermined amount of time if uncommon, hyperallergenic results occur, a step for administering topical antihistamine or anti-inflammatory if the patient is in discomfort after completion of the test, etc.

In an alternative embodiment, the instructions may also be provided online. In contrast to written instructions, a pamphlet, paper or portion of a kit container may include a printed website address to which the user (e.g., caretaker, doctor, nurse, etc.) is instructed to visit to find all relevant information and instructions for use of the skin test device.

Methods for use of a skin test device are also provided by the present disclosure. In an embodiment, a method for using a skin test device may include any or all of the following: removing a protective cover, impregnating or coating a substance of the skin test device with a material to be tested for the occurrence of an allergic reaction on a patient's skin, applying the skin test device to a portion of a patient's skin (e.g., an infant's back), allowing the skin test device to remain on the patient's skin for a predetermined amount of time, removing the skin test device after the expiration of the predetermined amount of time, marking any portion of the patient's skin that reacts to a material of the skin test device, documenting any skin patch reactions on the patient's skin, visiting a healthcare professional for interpretation of any skin patch reactions on the patient's skin, administering an antihistamine or an anti-inflammatory if the patient is in discomfort after completion of the test, cleansing the patch of skin corresponding to the test area, etc.

In an embodiment where the present skin test devices are applied for testing purposes and the results of the test are intended to be read/documented by an individual (e.g., caretaker, doctor, nurse, etc.) other than a doctor, the present disclosure provides for methods for allowing remote interpretation of the results by a doctor. For example, if a skin test device is applied to the skin of an infant and a parent or caregiver removes the device after a predetermined amount of time, the parent or caregiver can then document the region of skin corresponding to the previous locatation of the skin test device (e.g., by digital video, digital photograph, or the like) and send the documentation directly to the doctor. Thereafter, the doctor can interpret the results, and prescribe a prescription and/or assign a proper infant formula without ever having physically seen the test subject. Such a method can help to increase doctor office efficiency and reduce doctor office wait times for individuals who require physical examination by a doctor.

Alternatively, the results may easily be interpreted by a parent or caregiver if the parent or caregiver is educated in advance as to how to properly interpret the results. In this manner, a parent or caregiver may be able to easily determine which infant formula is the correct infant formula to assign to the infant, and may simply contact the doctor to confirm and/or inform the doctor of the assigned infant formula.

The methods of the present disclosure allow for stratification of patients with respect to specific infant formulas or protein components having different levels of allergic response. Accordingly, the methods of the present disclosure help a parent or caretaker to balance the physiological and financial implications of selecting a proper infant formula for consumption by an infant.

## Claims

1. A skin test device (10) comprising at least three chambers (24), **characterised in that**, a first chamber houses a negative control material, a second chamber houses extensively hydrolyzed protein infant formula or a partially hydrolyzed protein infant formula, and a third chamber houses an intact casein and whey protein infant formula.

2. The skin test device of Claim 1, wherein the extensively hydrolyzed protein infant formula comprises extensively hydrolyzed casein or extensively hydrolyzed whey protein, or wherein the partially hydrolyzed protein infant formula comprises partially hydrolyzed casein or partially hydrolyzed whey protein.

3. The skin test device of Claim 1, wherein the skin test device is a single, integral device.

4. The skin test device of Claim 1, wherein the skin test device comprises at least five individual chambers (24).

5. The skin test device of Claim 4, wherein at least four chambers (24) each houses a different infant formula and/or different protein component with respect to each other.

6. The skin test device of Claim 4, wherein a first chamber comprises saline, a second chamber comprises an extensively hydrolyzed protein infant formula, a third chamber comprises an intact casein and whey protein infant formula, a fourth chamber comprises a partially hydrolyzed protein infant formula, and a fifth chamber comprises an amino acid-based infant formula.

7. The skin test device of Claim 1, wherein the one of a negative control material, an extensively hydrolyzed protein infant formula, a partially hydrolyzed protein infant formula or an intact casein and whey protein infant formula is provided in a form selected from the group consisting of a liquid, a solid, a paste, a powder, a gel, and combinations thereof.

8. The skin test device of Claim 1, further comprising a substance housed by the chamber, the substance being impregnated or coated with the one of a negative control material, an extensively hydrolyzed protein infant formula, a partially hydrolyzed protein infant formula or an intact casein and whey protein infant formula.

9. A kit for skin testing, the kit comprising: the skin test device of claim 1, and instructions for applying the skin test device to the skin of an infant.

10. The kit of Claim 9, wherein the instructions comprise at least one of removing a removable cover (18) from the skin test device, adhering the skin test device to the skin of the infant, and allowing the skin test to remain adhered to the skin of the infant for a predetermined amount of time.

11. The kit of Claim 9, wherein the extensively hydrolyzed protein infant formula comprises extensively hydrolyzed casein or extensively hydrolyzed whey protein, or wherein the partially hydrolyzed protein infant formula comprises partially hydrolyzed casein or partially hydrolyzed whey protein.

12. The kit of Claim 9, wherein the skin test device further comprises a substance housed by each of the plurality of chambers, the substance being impregnated or coated with the one of a negative control material, an extensively hydrolyzed protein infant formula, a partially hydrolyzed protein infant formula or an intact casein and whey protein infant formula.

13. The kit of Claim 9, wherein a first chamber comprises saline, a second chamber comprises an extensively hydrolyzed protein infant formula, a third chamber comprises an intact casein and whey protein infant formula, a fourth chamber comprises a partially hydrolyzed protein infant formula, and a fifth chamber comprises an amino acid-based infant formula.

14. A method for using a skin test device, the method comprising: providing a parent or caretaker of the infant with the skin test device (10) of claim 1, and instructing the parent or caretaker to perform a skin patch test by adhering the skin test device to the skin of the infant for a predetermined amount of time.

15. The method of Claim 14 further comprising instructing the parent to schedule an appointment with a healthcare provider to read a result of the skin patch test after the predetermined amount of time, optionally further comprising instructing the parent to read a result of the skin patch test after removal of the skin test device.

16. The method of Claim 14 further comprising instructing the parent to remove the skin test device after the predetermined amount of time.

17. The method of Claim 14, wherein the extensively hydrolyzed protein infant formula comprises extensively hydrolyzed casein or extensively hydrolyzed whey protein, or wherein the partially hydrolyzed protein infant formula comprises partially hydrolyzed casein or partially hydrolyzed whey protein.

## Patentansprüche

1. Hauttestvorrichtung (10), umfassend mindestens drei Kammern (24), **dadurch gekennzeichnet, dass** eine erste Kammer ein Negativkontrollmaterial enthält, eine zweite Kammer eine Säuglingsformel mit weitgehend hydrolysiertem Protein oder eine Säuglingsformel mit teilweise hydrolysiertem Protein enthält und eine dritte Kammer eine Säuglingsformel mit unberührtem Casein und Molkeprotein enthält.

2. Hauttestvorrichtung nach Anspruch 1, wobei die Säuglingsformel mit weitgehend hydrolysiertem Protein weitgehend hydrolisiertes Casein oder weitgehend hydrolisiertes Molkeprotein umfasst, oder wobei die Säuglingsformel mit teilweise hydrolysiertem Protein teilweise hydrolisiertes Casein oder teilweise hydrolisiertes Molkeprotein umfasst.

3. Hauttestvorrichtung nach Anspruch 1, wobei die Hauttestvorrichtung eine einzelne, in sich geschlossene Vorrichtung ist.

4. Hauttestvorrichtung nach Anspruch 1, wobei die Hauttestvorrichtung mindestens fünf einzelne Kammern (24) umfasst.

5. Hauttestvorrichtung nach Anspruch 4, wobei mindestens vier Kammern (24) jeweils eine unterschiedliche Säuglingsformel und/oder eine unterschiedliche Proteinkomponente enthalten als die andere.

6. Hauttestvorrichtung nach Anspruch 4, wobei eine erste Kammer Kochsalzlösung umfasst, eine zweite Kammer eine Säuglingsformel mit weitgehend hydrolysiertem Protein umfasst, eine dritte Kammer eine Säuglingsformel mit unberührtem Casein und Molkeprotein umfasst, eine vierte Kammer eine Säuglingsformel mit teilweise hydrolysiertem Protein umfasst und eine fünfte Kammer eine Säuglingsformel auf Aminosäurebasis umfasst.

7. Hauttestvorrichtung nach Anspruch 1, wobei das eine eines Negativkontrollmaterials, einer Säuglingsformel mit weitgehend hydrolysiertem Protein, einer Säuglingsformel mit teilweise hydrolysiertem Protein oder einer Säuglingsformel mit unberührtem Casein und Molkeprotein in einer Form bereitgestellt wird, die aus der Gruppe ausgewählt ist, die aus einer Flüssigkeit, einem Feststoff, einer Paste, einem Pulver, einem Gel und Kombinationen davon besteht.

8. Hauttestvorrichtung nach Anspruch 1, ferner umfassend eine Substanz, die durch die Kammer enthalten ist, wobei die Substanz mit dem einem eines Negativkontrollmaterials, einer Säuglingsformel mit weitgehend hydrolysiertem Protein, einer Säuglingsformel mit teilweise hydrolysiertem Protein oder einer Säuglingsformel mit unberührtem Casein und Molkeprotein überzogen oder beschichtet ist.

9. Satz für Hauttests, der Satz umfassend: die Hauttestvorrichtung nach Anspruch 1 und Anweisungen zum Anwenden der Hauttestvorrichtung auf der Haut eines Säuglings.

10. Satz nach Anspruch 9, wobei die Anweisungen mindestens eines von Entfernen einer abnehmbaren Abdeckung (18) von der Hauttestvorrichtung, Aufkleben der Hauttestvorrichtung auf die Haut des Säuglings und Verbleibenlassen des Hauttests auf der Haut des Säuglings für eine vorbestimmte Zeitspanne umfassen.

11. Satz nach Anspruch 9, wobei die Säuglingsformel mit weitgehend hydrolysiertem Protein weitgehend hydrolysiertes Casein oder weitgehend hydrolysiertes Molkeprotein umfasst, oder wobei die Säuglingsformel mit teilweise hydrolysiertem Protein teilweise hydrolysiertes Casein oder teilweise hydrolysiertes Molkeprotein umfasst.

12. Satz nach Anspruch 9, wobei die Hauttestvorrichtung ferner eine Substanz umfasst, die durch jede der Vielzahl von Kammern enthalten ist, wobei die Substanz mit dem einem eines Negativkontrollmaterials, einer Säuglingsformel mit weitgehend hydrolysiertem Protein, einer Säuglingsformel mit teilweise hydrolysiertem Protein oder einer Säuglingsformel mit unberührtem Casein und Molkeprotein überzogen oder beschichtet ist.

13. Satz nach Anspruch 9, wobei eine erste Kammer Kochsalzlösung umfasst, eine zweite Kammer eine Säuglingsformel mit weitgehend hydrolysiertem Protein umfasst, eine dritte Kammer eine Säuglingsformel mit unberührtem Casein und Molkeprotein umfasst, eine vierte Kammer eine Säuglingsformel mit teilweise hydrolysiertem Protein umfasst und eine fünfte Kammer eine Säuglingsformel auf Aminosäurebasis umfasst.

14. Verfahren zum Verwenden einer Hauttestvorrichtung, das Verfahren umfassend: Bereitstellen der Hauttestvorrichtung (10) nach Anspruch 1 für ein Elternteil oder einen Betreuer des Säuglings und Anweisen des Elternteils oder Betreuers, einen Hautepikutantest durch Kleben der Hauttestvorrichtung für eine vorbestimmte Zeitspanne auf die Haut des Säuglings durchzuführen.

15. Verfahren nach Anspruch 14, ferner umfassend das Anweisen des Elternteils, einen Termin mit einem Gesundheitsdienstleister zu vereinbaren, um ein Ergebnis des Hautpepikutantests nach der vorbestimmten Zeitspanne abzulesen, und optional ferner umfassend das Anweisen des Elternteils, ein Ergebnis des Hautepikutantests nach der Entfernung der Hauttestvorrichtung abzulesen.

16. Verfahren nach Anspruch 14, ferner umfassend das Anweisen des Elternteils, die Hauttestvorrichtung nach der vorbestimmten Zeitspanne zu entfernen.

17. Verfahren nach Anspruch 14, wobei die Säuglingsformel mit weitgehend hydrolysiertem Protein weitgehend hydrolysiertes Casein oder weitgehend hydrolysiertes Molkeprotein umfasst, oder wobei die Säuglingsformel mit teilweise hydrolysiertem Protein teilweise hydrolysiertes Casein oder teilweise hydrolysiertes Molkeprotein umfasst.

## Revendications

1. Dispositif de test cutané (10) comprenant au moins trois chambres (24), **caractérisé en ce que,** une première chambre loge un matériau témoin négatif, une deuxième chambre loge une préparation pour nourrissons de protéine fortement hydrolysée ou une préparation pour nourrissons de protéine partiellement hydrolysée, et une troisième chambre loge une préparation pour nourrissons de caséine et de protéine de lactosérum intactes.

2. Dispositif de test cutané selon la revendication 1, dans lequel la préparation pour nourrissons de protéine fortement hydrolysée comprend de la caséine fortement hydrolysée ou de la protéine de lactosérum fortement hydrolysée, ou dans lequel la préparation pour nourrissons de protéine partiellement hydrolysée comprend de la caséine partiellement hydrolysée ou de la protéine de lactosérum partiellement hydrolysée.

3. Dispositif de test cutané selon la revendication 1, dans lequel le dispositif de test cutané est un dispositif unique, intégral.

4. Dispositif de test cutané selon la revendication 1, dans lequel le dispositif de test cutané comprend au moins cinq chambres individuelles (24).

5. Dispositif de test cutané selon la revendication 4, dans lequel au moins quatre chambres (24) logent chacune une préparation pour nourrissons différente et/ou un composant protéique différent les unes par rapport aux autres.

6. Dispositif de test cutané selon la revendication 4, dans lequel une première chambre comprend une solution saline, une deuxième chambre comprend une préparation pour nourrissons de protéine fortement hydrolysée, une troisième chambre comprend une préparation pour nourrissons de caséine et de protéine de lactosérum intactes, une quatrième chambre comprend une préparation pour nourrissons de protéine partiellement hydrolysée, et une cinquième chambre comprend une préparation pour nourrissons à base d'acides aminés.

7. Dispositif de test cutané selon la revendication 1, dans lequel l'un parmi un matériau témoin négatif, une préparation pour nourrissons de protéine fortement hydrolysée, une préparation pour nourrissons de protéine partiellement hydrolysée ou une préparation pour nourrissons de protéine de lactosérum et de caséine intactes est fourni sous une forme choisie dans le groupe constitué d'un liquide, d'un solide, d'une pâte, d'une poudre, d'un gel, et des combinaisons de ceux-ci.

8. Dispositif de test cutané selon la revendication 1, comprenant en outre une substance logée par la chambre, la substance étant imprégnée ou revêtue de l'un parmi un matériau témoin négatif, une préparation pour nourrissons de protéine fortement hydrolysée, une préparation pour nourrissons de protéine partiellement hydrolysée ou une préparation pour nourrissons de protéine de lactosérum et de caséine intactes.

9. Kit pour test cutané, le kit comprenant : le dispositif de test cutané selon la revendication 1, et des instructions pour appliquer le dispositif de test cutané à la peau d'un nourrisson.

10. Kit selon la revendication 9, dans lequel les instructions comprennent au moins l'une parmi retirer un couvercle amovible (18) du dispositif de test cutané, faire adhérer le dispositif de test cutané à la peau du nourrisson, et permettre au test cutané de rester collé à la peau du nourrisson pendant un laps de temps prédéterminé.

11. Kit selon la revendication 9, dans lequel la préparation pour nourrissons de protéine fortement hydrolysée comprend de la caséine fortement hydrolysée ou de la protéine de lactosérum fortement hydrolysée, ou dans lequel la préparation pour nourrissons de protéine partiellement hydrolysée comprend de la caséine partiellement hydrolysée ou de la protéine de lactosérum partiellement hydrolysée.

12. Kit selon la revendication 9, dans lequel le dispositif de test cutané comprend en outre une substance logée par chacune de la pluralité de chambres, la substance étant imprégnée ou revêtue de l'un parmi un matériau témoin négatif, une préparation pour nourrissons de protéine fortement hydrolysée, une préparation pour nourrissons de protéine partiellement hydrolysée ou une préparation pour nourrissons de protéine de lactosérum et de caséine intactes.

13. Kit selon la revendication 9, dans lequel une première chambre comprend une solution saline, une deuxième chambre comprend une préparation pour nourrissons de protéine fortement hydrolysée, une troisième chambre comprend une préparation pour nourrissons de caséine et de protéine de lactosérum intactes, une quatrième chambre comprend une préparation pour nourrissons de protéine partiellement hydrolysée, et une cinquième chambre comprend une préparation pour nourrissons à base d'acides aminés.

14. Procédé d'utilisation d'un dispositif de test cutané, le procédé comprenant : la fourniture à un parent ou à un soignant du nourrisson du dispositif de test cutané (10) selon la revendication 1, et l'instruction au parent ou au soignant d'effectuer un test épicutané en adhérant le dispositif de test cutané à la peau du nourrisson pendant un laps de temps prédéterminé.

15. Procédé selon la revendication 14, comprenant en outre l'instruction au parent de planifier un rendez-vous avec un fournisseur de soins de santé pour lire un résultat du test épicutané après le laps de temps prédéterminé, comprenant en outre facultativement l'instruction au parent de lire un résultat du test épicutané après le retrait du dispositif de test cutané.

16. Procédé selon la revendication 14, comprenant en outre l'instruction au parent de retirer le dispositif de test cutané après le laps de temps prédéterminé.

17. Procédé selon la revendication 14, dans lequel la préparation pour nourrissons de protéine fortement hydrolysée comprend de la caséine fortement hydrolysée ou de la protéine de lactosérum fortement hydrolysée, ou dans lequel la préparation pour nourrissons de protéine partiellement hydrolysée comprend de la caséine partiellement hydrolysée ou de la protéine de lactosérum partiellement hydrolysée.
